# EUROPÄISCHE PATENTSCHRIFT

(11) **EP 1 043 387 B1**
(45) Veröffentlichungstag und Bekanntmachung des Hinweises auf die Patenterteilung: **08.06.2005**
(21) Anmeldenummer: 00105813.0
(22) Anmeldetag: 18.03.2000
(51) Int. Cl.: C11D 3/34, C11D 17/06, C11D 1/22, C11D 11/02, C07C 309/31

(54) **Alkylbenzolsulfonat-Granulate**
Alkylbenzenesulfonate granulates
Granules d'alkylbenzènesulfonate

(30) Priorität: 27.03.1999 DE 29905721 U
(43) Veröffentlichungstag der Anmeldung: 11.10.2000
(73) Patentinhaber: Cognis Deutschland GmbH & Co. KG, 40589 Düsseldorf (DE)
(72) Erfinder: Kischkel, Ditmar, 40789 Monheim (DE); Schmid, Karl-Heinz Dr., 40822 Mettmann (DE); Syldath, Andreas Dr., 40789 Monheim (DE); Blochwitz, Olaf, 39307 Genthin (DE); Tesmann, Holger Dr., 41363 Jüchen (DE)

(56) Entgegenhaltungen:
- WO-A-94/18303
- DE-A- 2 452 414
- DE-B- 1 004 323
- GB-A- 2 323 848
- US-A- 3 726 813
- DATABASE CAPLUS [Online] CHEMICAL ABSTRACTS SERVICE, COLUMBUS, OHIO, US; retrieved from STN Database accession no. 1975:430329 XP002228524 & ES 397 948 A (SOCIEDAD ANON CAMP FABRICA DE JABONES) 1. Juli 1974 (1974-07-01)

## Beschreibung

Die Erfindung betrifft staubfreie, nicht-klebende Alkylbenzolsulfonat-Granulate mit hohem Schüttgewicht, die Salze der Toluolsulfonsäure enthalten. Derartige Granulate sind zur Herstellung fester Wasch-, Spül- und Reinigungsmittel bestens geeignet.

Tensidgranulate mit hohen Schüttgewichten sind beispielsweise gemäß der deutschen Patentanmeldung **DE-A- 41 27 323** zugänglich, wobei die Tensidgranulate durch eine Granulierung in der Wirbelschicht hergestellt werden. Dazu wird eine Tensid-Zubereitungsform, die eine nicht-tensidische Flüssigkomponente (in der Regel Wasser) aufweist, gewünschtenfalls unter Zumischung eines anorganischen oder organischen Feststoffes granuliert und gleichzeitig getrocknet. Die Tensid-Zubereitungsform kann dabei als Tenside C₉-C₁₃-Alkylbenzolsulfonate enthalten.

Aus der deutschen Patentanmeldung **DE-A 42 32 874** ist ein analoges Granulierverfahren in der Wirbelschicht bekannt, wobei Aniontensidgranulate durch Neutralisation von Aniontensiden in ihrer Säureform dadurch erhalten werden, daß die Aniontenside in ihrer Säureform mit einem pulverförmigen Neutralisationsmittel neutralisiert, dabei gleichzeitig granuliert und die entstehenden Granulate gewünschtenfalls gleichzeitig getrocknet werden. Wiederum kann dabei als anionisches Tensid Alkylbenzolsulfonat in seiner Säureform allein oder im Tensidgemisch eingesetzt werden.

Die genannten Verfahren führen zu hochkonzentrierten Tensidgranulaten mit vorzugsweise erhöhtem Schüttgewicht. Gerade bei der Verarbeitung niedrig schmelzender und schlecht kristallisierender Aniontenside, wie von Alkylbenzolsulfonaten, treten jedoch Probleme bezüglich der Rieselfähigkeit bzw. der Klebrigkeit der Granulate auf.

Dieser Nachteil kann gemäß der deutschen Offenlegungsschrift **DE-A- 43 04 015** behoben werden, wenn die Granulate zum Schutz vor dem Verklumpen nach dem Austritt aus der Wirbelschicht im Abzugsluftstrom mit einem Stabilisator beschichtet werden (Abpuderung). Als Granulatstabilisatoren werden Wachse, Zeolithe, Alkalicarbonate, Alkalisulfate, Alkalisilikate, Polyethylenglykole, alkoxylierte Alkohole, Silikone oder auch Aniontenside in ihrer Säureform vorgeschlagen. Nach diesem Verfahren können Granulate von Alkylbenzolsulfonaten mit Aktivsubstanzgehalten um die 30 Gew.-% erhalten werden.

Granulate von Alkylbenzolsulfonaten mit höheren Aktivsubstanzgehalten bis zu etwa 55 Gew.-% können gemäß der internationalen Anmeldung **WO 94/18303** auch erhalten werden, wenn gemäß der Methode der Sprühneutralisation anionische Tenside in Säureform und alkalische Lösungen getrennt in den Granulier- und Trocknungsraum versprüht werden und die anschließende Granulierung unter Zumischung fester anorganischer oder organischer Stoffe erfolgt. Derartige Feststoffe wirken als Träger, die die Verklumpbarkeit der Tensidgranulate reduzieren. Als geeignete Feststoffe werden in der Anmeldung Alkalicarbonate, Alkalisulfate, Zeolithe und Citrate genannt.

. Auf dem Markt werden aber Alkylbenzolsulfonat-Granulate mit höheren Aktivsubstanzgehalten gefordert, die zudem möglichst frei von Zeolithen sein sollen, damit der Waschmittelhersteller größere Freiheiten bei der Rezepturzusammenstellung hat.

US-A-3 726 813 beschreibt extrudierte Tensidgranulate enthaltend Alkylbenzolsulfonat und C₁₋₃-Alkylbenzolsulfonat. ES-A-397 948 beschreibt granulate nichtklebrige Reinigungsmittel hergestellt durch kosulfonierung von Alkylbenzol und Toluol.

Zur Zeit sind auf dem Markt Pulver von Alkylbenzolsulfonaten in Mischung mit bis zu etwa 10 Gew.-% Toluolsulfonat erhältlich, aber diese weisen jedoch nur Schüttgewichte von maximal 500 g/l auf und sind aufgrund des Feinanteils der Pulver mit Teilchengrößen unter 0,5 mm staubend.

DE-A-2 452 414 und DE-B-1 004 323 beschreiben rieselfähige, nichtklebrige Reinigungsmittelpulver enthaltend Toluolsulfonat und Alkylbenzolsulfonat.

Demnach hat die komplexe Aufgabe der vorliegenden Erfindung darin bestanden, Granulate von Alkylbenzolsulfonaten mit hohen Schüttgewichten über 550 g/l und hohen Aktivsubstanzgehalten, insbesondere über 60 Gew.-% - bezogen auf Granulat - zur Verfügung zu stellen, die nicht kleben bzw. zum Verklumpen neigen. Des weiteren sollten die Granulate möglichst frei von Zeolithen sein. Und schließlich sollten die Granulate staubfrei sein, d.h. keinen bzw. einen vernachlässigbar kleinen Anteil an Granulaten mit Korngrößen (bzw. Teilchendurchmessern) unter 0,5 mm aufweisen.

Die Aufgabe der vorliegenden Erfindung konnte gelöst werden, wenn die Granulate der Alkylbenzolsulfonate Salze der Toluolsulfonsäure enthalten.

Ein Gegenstand der vorliegenden Erfindung betrifft daher nichtklebrige, staubfreie Alkylbenzolsulfonat-Granulate, wobei die Alkylbenzolsulfonate 9 bis 18 Kohlenstoffatomen im Alkylrest enthalten, enthaltend Toluolsulfonate, mit Aktivsubstanzgehalten von mind. 60 Gew.-% und Schüttgewichten über 550 g/l, wobei die Granulate zu mindestens 50 Gew.-% nach der Siebanalyse einen Teilchendurchmesser von mind. 0,8 mm aufweisen und die Korngrößeverteilung der Granulate wie folgt ist:

| Teilchengröße | prozentuale Zusammenseztung in Gew.-% |
|---|---|
| unter 0,4 mm | 0 - 3 % |
| zwischen 0,4 und 0,8 mm | 22 - 35 % |
| zwischen 0,8 mm und 1,25 mm | 40 - 60 % |
| zwischen 1,25 und 1,6 mm | 10 - 25 % |
| über 1,6 mm | 0 - 0,5 % |

Alkylbenzolsulfonate im Sinne der Erfindung haben 9 bis 18 Kohlenstoffatome im Alkylrest und folgen vorzugsweise der Formel **(I),**

**R**^{**1**}**-Ph-SO**_{**3**}**X (I)**

in der R¹ für einen verzweigten, vorzugsweise jedoch linearen Alkylrest mit 9 bis 18 Kohlenstoffatomen, Ph für einen Phenylrest und X für ein Alkali- und/oder Erdalkalimetall, Ammonium, Alkylammonium, Alkanolammonium oder Glucammonium steht. Bevorzugt werden Dodecylbenzolsulfonate, Tetradecylbenzolsulfonate, Hexadecylbenzolsulfonate sowie deren technische Gemische in Form der Natriumsalze.

Die erfindungsgemäßen Granulate enthalten die Alkylbenzolsulfonate mindestens in Mengen von 60 Gew.-%, d.h. der Aktivsubstanzgehalt der Granulate beträgt mindestens 60 Gew.-%, vorzugsweise sind sie in Mengen von 70 bis 90 Gew.-% und insbesondere in Mengen von etwa 75 bis 85 Gew.-% - bezogen auf Granulat - enthalten.

Des weiteren enthalten die erfindungsgemäßen Granulate zwingend Salze der Toluolsulfonsäure, insbesondere das Natriumsalz. Die Toluolsulfonate sind vorzugsweise in Mengen von 3 bis 10, bevorzugt von 4 bis 8 Gew.-% - bezogen auf Granulat - enthalten. Vorteilhafterweise sind die Toluolsulfonate zumindest anteilsweise in die Granulate eingebaut. Die Salze der Toluolsulfonate können in reiner oder technischer Qualität enthalten sein. Sofern die technische Qualität enthalten ist, sind Spuren an Cumolsulfonat bis zu ca. 20 Gew.-% - bezogen auf Toluolsulfonat-Gehalt - möglich.

Die erfindungsgemäßen Granulate können noch in Mengen bis zu 5 Gew.-%, vorzugsweise bis 3 Gew.-% und insbesondere von 1,5 bis 2,5 Gew.-% Restmengen an Wasser enthalten, die durch das Herstellverfahren bedingt sind.

Zusätzlich können die erfindungsgemäßen Granulate noch in Mengen ad 100 Gew.-% Alkalisalze, vorzugsweise Alkalicarbonate und/oder Alkalisulfate, insbesondere deren Natriumsalze enthalten.

Die erfindungsgemäßen Granulate werden vorzugsweise hergestellt durch Neutralisation von Alkylbenzolsulfonsäuren (Säureform) mit einem Neutralisationsmittel unter gleichzeitiger Granulierung und Trocknung in der Wirbelschicht und in Anwesenheit von Toluolsulfonsäure bzw. deren Salze in den beschriebenen Mengen. Die Neutralisation der mit einem festen Neutralisationsmittel, beispielsweise Alkalihydroxide oder Alkalicarbonate, kann beispielsweise gemäß der schon zitierten deutschen Offenlegungsschrift **DE-A- 42 32 874** erfolgen. Besonders bevorzugt erfolgt die Neutralisation der Alkylbenzolsulfonsäure jedoch mittels einer wäßrigen alkalischen Lösung, insbesondere von Alkalihydroxiden, nach dem Prinzip der Sprühneutralisation, die analog der schon zitierten internationalen Anmeldung **WO 94/18303** erfolgen kann. Danach wird die Alkylbenzolsulfonsäure mit einem gasförmigen Medium beaufschlagt und über eine Mehrstoff-Düse, beispielsweise über eine 2-Stoff -Düse in den Granulier- und Trocknungsraum versprüht. Ebenso wird eine wäßrige alkalische Lösung mit einem gasförmigen Medium beaufschlagt und gleichzeitig mit der Alkylbenzolsulfonsäure in nahezu stöchiometrischen Mengen über eine weitere Mehrstoff-Düse in den Granulier- und Trocknungsraum versprüht. In diesem Fall findet die Neutralisation und damit die Herstellung der Alkylbenzolsulfonate direkt in dem Granulierund Trocknungsraum statt. Der Begriff "nahezu stöchiometrische Mengen" bedeutet dabei, daß das Verhältnis der Anzahl saurer Gruppen zu alkalischen Gruppen vorzugsweise im Bereich von 1,1 : 1 bis 0,8 : 1 und insbesondere von 1 : 1 bis 0,9 : 1 liegt.

Gemäß einer bevorzugten Verfahrensausgestaltung werden die getrennt mit einem gasförmigen Medium beaufschlagten sauren und alkalischen Reaktantenströme über eine einzige Mehrstoff-Düse, beispielsweise über eine 2- oder 3-Stoff-Düse, zusammengeführt und in den Granulier- und Trocknungsraum versprüht. In diesem Fall erfolgt die Neutralisation im wesentlichen Umfang in der Düse bzw. direkt beim Austritt aus der Düse. In der praktischen Durchführung werden die Ausgangsstoffe beispielsweise unter Verwendung von Kolbenpumpen nahezu stöchiometrisch dosiert in eine bzw. zwei nicht weiter modifizierte handelsübliche Düse(n) bzw. ein oder zwei Sprührohr(e) eingebracht. Unmittelbar vor der Düse erfolgt das Zumischen des gasförmigen Mediums (Treibgas). Im Falle des Versprühens der zusammengeführten Reaktantenströme bedeutet dies, daß das Zumischen des gasförmigen Mediums in die Reaktantenströme vor deren Zusammentreffen vor bzw. in der Düse erfolgt. Weitere technische Einzelheiten zur Durchführung des Verfahrens, beispielsweise bezüglich der Steuerung der Reaktionstemperatur, der Strömungsgeschwindigkeit der Reaktantenströme oder des Drucks, bei dem das Reaktionsgemisch in den Granulier- und Trocknungsraum versprüht wird, können der europäischen Patentanmeldung **EP 0 319 819** entnommen werden.

Die Neutralisationsreaktion wird vorzugsweise mit konzentrierten wäßrigen alkalischen Lösungen, beispielsweise Lösungen von Hydroxiden, Carbonaten oder Hypochloriten des Natriums oder Kaliums, insbesondere mit einer konzentrierten wäßrigen Natronlauge und/oder Kalilauge durchgeführt, wobei Konzentrationen um 45 bis 55 Gew.-% besonders bevorzugt sind.

Als gasförmiges Medium (Treibgas) eignen sich insbesondere gegenüber den Ausgangs- und Endstoffen inerte Gase wie Luft oder Stickstoff.

Die versprühten Reaktantenströme an Alkylbenzolsulfonsäure und flüssigem Neutralisationsmittel werden nun gleichzeitig granuliert und getrocknet. Dabei wird unter "Trocknung" das teilweise oder vollständige Entfernen von Wasser verstanden. Falls gewünscht, können Restwerte an freiem, das heißt nicht-gebundenem Wasser vorhanden sein, solange die fertigen Granulate rieselfähig und nicht klebend sind. Vorzugsweise wird jedoch der Gehalt an freiem Wasser der Granulate (Restmengen an Wasser) unter 5 Gew.-% und insbesondere zwischen 1,5 und 2,5 Gew.-%, jeweils bezogen auf die fertigen erfindungsgemäßen Granulate, betragen.

Nach einer weiteren Möglichkeit können die erfindungsgemäßen Granulate hergestellt werden, indem wäßrige Pasten von Alkalisalzen der Alkylbenzolsulfonsäure direkt eingesprüht werden.

Die Herstellung der erfindungsgemäßen Granulate erfolgt vorzugsweise in einer kontinuierlich laufenden Wirbelschicht. Dabei ist es möglich, über eine Feststoffdosierung feste Toluolsulfonsäure bzw. bevorzugt die Salze der Toluolsulfonsäure gleichzeitig und separat zuzusetzen, wobei diese pneumatisch über Blasleitungen unterhalb der Sprühdüsen, aber oberhalb der sich ausbildenden Wirbelschicht, zudosiert werden. Es ist auch möglich, wenn auch nicht bevorzugt, die Toluolsulfonate als wäßrige Lösung über Sprühdüsen in die Wirbelschicht einzubringen.

Typisch eingesetzte Wirbelschicht-Apparate besitzen Bodenplatten mit Abmessungen von mindestens 0,4 m, in der Regel mit einem Durchmesser zwischen 0,4 und 5 m, beispielsweise 1,5 m oder 2,6 m. Es sind jedoch auch Wirbelschicht-Apparate geeignet, die eine Bodenplatte mit einem größeren Durchmesser als 5 m aufweisen. Als Bodenplatte wird vorzugsweise eine Lochbodenplatte, ein Sandwichboden oder eine Conidurplatte (Handelsprodukt der Firma Hein & Lehmann, Bundesrepublik Deutschland) eingesetzt. In der Regel erfolgt das Verfahren bei Wirbelluftgeschwindigkeiten zwischen 1 und 8 m/s und insbesondere zwischen 1,5 und 5,5 m/s. Der Austrag der Granulate aus der Wirbelschicht erfolgt vorteilhafterweise über eine Größenklassierung der Granulate. Diese Klassierung kann beispielsweise mittels einer Siebvorrichtung oder durch einen entgegengeführten Luftstrom (Sichterluft) erfolgen, der so reguliert wird, daß erst Teilchen ab einer bestimmten Teilchengröße aus der Wirbelschicht entfernt und kleinere Teilchen in der Wirbelschicht zurückgehalten werden. Somit setzt sich die einströmende Luft aus der Sichterluft und der Bodenluft zusammen, wobei beide entweder beheizt oder unbeheizt bzw. eine beheizt und die andere unbeheizt sein können. In Ausnahmefällen kann auch eine gekühlte Bodenluft eingesetzt werden. Die Bodenlufttemperatur liegt dabei im allgemeinen zwischen - 20 und 400 °C, vorzugsweise zwischen 35 und 350 °C und insbesondere zwischen 35 und 120 °C. Vorteilhafterweise beträgt die Temperatur der Wirbelluft etwa 5 cm oberhalb der Bodenplatte 10 bis 120 °C, vorzugsweise 20 bis 90 °C und insbesondere 30 bis 85 °C. Die Luftaustrittstemperatur ergibt sich zwangsläufig aus den Reaktionsbedingungen. Bei dem Verfahren in der Wirbelschicht ist es notwendig, daß zu Beginn des Verfahrens eine Startmasse vorhanden ist, die als anfänglicher Träger für die eingesprühte Tensid-Zubereitungsform dient. Als Startmasse eignen sich vor allem solche Tensidgranulate, die bereits bei einem vorangegangenen Verfahrensablauf erhalten wurden.

In der Wirbelschicht verdampft das Wasser teilweise oder vollständig und es entstehen angetrocknete bis getrocknete Keime, die mit weiteren Mengen eingebrachter Tensid-Zubereitungsform umhüllt, granuliert und wiederum gleichzeitig getrocknet werden. Durch Eintrag der Toluolsulfonate in die Wirbelschicht werden zum einen diese in die Alkylbenzolsulfonat-Tensidkeime bzw. Granulate eingebaut und zum anderen umhüllen sie die Granulate.

Die erfindungsgemäßen Alkylbenzolsulfonat-Granulate weisen im Gegensatz zu handelsüblichen Pulvern ein hohes Schüttgewicht auf, das über 550 g/l, vorzugsweise zwischen 550 und 650 g/l liegt. Außerdem haben die erfindungsgemäßen Granulate im Gegensatz zu handelsüblichen Pulvern eine andere Kornverteilung, so weisen die erfindungsgemäßen Granulate zu über 50 % nach der Siebanalyse einen Teilchendurchmesser von mindestens 0,8 mm auf. Kornverteilungen der erfindungsgemäßen Granulate nach der Siebanalyse sind:

| Teilchengröße | prozentuale Zusammenseztung in Gew.-% |
|---|---|
| unter 0,4 mm | 0 - 3 % |
| zwischen 0,4 und 0,8 mm | 22 - 35 % |
| zwischen 0,8 mm und 1,25 mm | 40 - 60 % |
| zwischen 1,25 und 1,6 mm | 10 - 25 % |
| über 1,6 mm | 0 - 0,5 %. |

Schließlich lassen sich die erfindungsgemäßen Granulate aufgrund des enthaltenen Toluolsulfonats besser und schneller trocknen, so daß geringere Verweilzeiten in der Wirbelschicht resultieren bzw. bei gleicher Verweilzeit ein geringerer Wassergehalt erzielt werden kann als ohne den Zusatz an Toluolsulfonat. Ein weiterer Vorteil der erfindungsgemäßen Granulate ist, daß sie aufgrund des Toluolsulfonats nicht klebrig sind. Sogesehen wirkt Toluolsulfonat als Abpuderungsagenz und als Beschleuniger der Trocknung.

Ein weiterer Gegenstand der vorliegenden Erfindung betrifft feste Wasch-, Spül- und Reinigungsmittel enthaltend Alkylbenzolsulfonat-Granulate mit Aktivsubstanzgehalten von mindestens 60 Gew.-% und Schüttgewichten über 550 g/l enthaltend Toluolsulfonate. Die festen Wasch-, Spül- und Reinigungsmittel können zusätzlich die bekannten weiteren Inhaltsstoffe enthalten.

Bekannte weitere Inhaltsstoffe sind beispielsweise anionische, nichtionische, kationische und/oder amphotere bzw. amphotere Tenside. Typische Beispiele für **anionische Tenside** sind Seifen, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere** bzw. **zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.),** **"Katalysatoren, Tenside und Mineralöladditive", Thieme Verlag, Stuttgart, 1978, S. 123-217** verwiesen.

Die festen Wasch-, Spül- und Reinigungsmittel können neben den genannten noch weitere typische Inhaltsstoffe, wie beispielsweise Builder, Bleichmittel, Bleichaktivatoren, Waschkraftverstärker, Enzyme, Enzymstabilisatoren, Vergrauungsinhibitoren, optische Aufheller, Soil repellants, Schauminhibitoren, anorganische Salze sowie Duft- und Farbstoffe enthalten.

Als feste **Builder** wird insbesondere feinkristalliner, synthetisches und gebundenes Wasser enthaltender Zeolith wie Zeolith NaA in Waschmittelqualität eingesetzt. Geeignet sind jedoch auch Zeolith NaX sowie Mischungen aus NaA und NaX. Der Zeolith kann als sprühgetrocknetes Pulver oder auch als ungetrocknete, von ihrer Herstellung noch feuchte, stabilisierte Suspension zum Einsatz kommen. Für den Fall, daß der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen oder ethoxylierte Isotridecanole. Geeignete Zeolithe weisen eine mittlere Teilchengröße von weniger als 10 µm (Volumenverteilung; Meßmethode: Coulter Counter) auf und enthalten vorzugsweise 18 bis 22, insbesondere 20 bis 22 Gew.-% an gebundenem Wasser. Geeignete Substitute bzw. Teilsubstitute für Zeolithe sind kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung **EP 0164514 A** beschrieben. Bevorzugte kristalline Schichtsilicate sind solche, in denen M in der allgemeinen Formel für Natrium steht und x die Werte 2 oder 3 annimmt. Insbesondere sind sowohl β- als auch γ-Natriumdisilicate Na₂Si₂O₅·yH₂O bevorzugt, wobei β-Natriumdisilicat beispielsweise nach dem Verfahren erhalten werden kann, das in der internationalen Patentanmeldung **WO 91/08171** beschrieben ist. Die erfindungsgemäßen Pulverwaschmittel enthalten als feste Builder vorzugsweise 10 bis 60 Gew.-% Zeolith und/oder kristalline Schichtsilicate, wobei Mischungen von Zeolith und kristallinen Schichtsilicaten in einem beliebigen Verhältnis besonders vorteilhaft sein können. Insbesondere ist es bevorzugt, daß die Mittel 20 bis 50 Gew.-% Zeolith und/oder kristalline Schichtsilicate enthalten. Besonders bevorzugte Mittel enthalten bis 40 Gew.-% Zeolith und insbesondere bis 35 Gew.-% Zeolith, jeweils bezogen auf wasserfreie Aktivsubstanz. Weitere geeignete Inhaltsstoffe der Mittel sind wasserlösliche amorphe Silicate; vorzugsweise werden sie in Kombination mit Zeolith und/oder kristallinen Schichtsilicaten eingesetzt. Insbesondere bevorzugt sind dabei Mittel, welche vor allem Natriumsilicat mit einem molaren Verhältnis (Modul) Na₂O : SiO₂ von 1:1 bis 1:4,5, vorzugsweise von 1:2 bis 1:3,5, enthalten. Der Gehalt der Mittel an amorphen Natriumsilicaten beträgt dabei vorzugsweise bis 15 Gew.-% und vorzugsweise zwischen 2 und 8 Gew.-%. Auch Phosphate wie Tripolyphosphate, Pyrophosphate und Orthophosphate können in geringen Mengen in den Mitteln enthalten sein. Vorzugsweise beträgt der Gehalt der Phosphate in den Mitteln bis 15 Gew.-%, jedoch insbesondere 0 bis 10 Gew.-%. Außerdem können die Mittel auch zusätzlich Schichtsilicate natürlichen und synthetischen Ursprungs enthalten. Derartige Schichtsilicate sind beispielsweise aus den Patentanmeldungen **DE 2334899 B, EP 0026529 A** und **DE 3526405 A** bekannt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilicate, die zur Gruppe der mit Wasser quellfähigen Smectite zählen, sind z.B. solche der allgemeinen Formeln

(OH)₄Si_{8-y}Al_{y}(MgₓAl₄₋ₓ)O₂₀ Montmorrilonit

(OH)₄Si_{8-y}Al_{y}(Mg_{6-z}Li_{z})O₂₀ Hectorit

(OH)₄Si_{8-y}Al_{y}(Mg_{6-z} Al_{z})O₂₀ Saponit

mit x = 0 bis 4, y = 0 bis 2, z = 0 bis 6. Zusätzlich kann in das Kristallgitter der Schichtsilicate gemäß den vorstehenden Formeln geringe Mengen an Eisen eingebaut sein. Ferner können die Schichtsilicate aufgrund ihrer ionenaustauschenden Eigenschaften Wasser-stoff-, Alkali-, Erdalkaliionen, insbesondere Na⁺ und Ca²⁺ enthalten. Die Hydratwassermenge liegt meist im Bereich von 8 bis 20 Gew.-% und ist vom Quellzustand bzw. von der Art der Bearbeitung abhängig. Brauchbare Schichtsilicate sind beispielsweise aus **US 3,966,629, US 4,062,647, EP 0026529 A** und **EP 0028432 A** bekannt. Vorzugsweise werden Schichtsilicate verwendet, die aufgrund einer Alkalibehandlung weitgehend frei von Calciumionen und stark färbenden Eisenionen sind. Brauchbare organische Gerüstsubstanzen sind beispielsweise die bevorzugt in Form ihrer Natriumsalze eingesetzten Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Geeignete polymere Polycarboxylate sind beispielsweise die Natriumsalze der Polyacrylsäure oder der Polymethacrylsäure, beispielsweise solche mit einer relativen Molekülmasse von 800 bis 150.000 (auf Säure bezogen). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5.000 bis 200.000, vorzugsweise 10.000 bis 120.000 und insbesondere 50.000 bis 100.000. Der Einsatz polymerer Polycarboxylate ist nicht zwingend erforderlich. Falls jedoch polymere Polycarboxylate eingesetzt werden, so sind Mittel bevorzugt, welche biologisch abbaubare Polymere, beispielsweise Terpolymere, die als Monomere Acrylsäure und Maleinsäure bzw. deren Salze sowie Vinylalkohol bzw. Vinylalkohol-Derivate oder die als Monomere Acrylsäure und 2-Alkylallylsulfonsäure bzw. deren Salze sowie Zuckerderivate enthalten. Insbesondere sind Terpolymere bevorzugt, die nach der Lehre der deutschen Patentanmeldungen **DE 4221381 A** und **DE 4300772 A** erhalten werden. Weitere geeignete Buildersubstanzen sind Polyacetale, welche durch Umsetzung von Dialdehyden mit Polyolcarbonsäuren, welche 5 bis 7 Kohlenstoffatome und mindestens 3 Hydroxylgruppen aufweisen, beispielsweise wie in der europäischen Patentanmeldung **EP 0280223 A** beschrieben erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Unter den als **Bleichmittel** dienenden, in Wasser Wasserstoffperoxid liefernden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie Salze der Persäuren, wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können, trägt es zur Erhöhung der Stabilität des Mittels bei.

Um beim Waschen bei Temperaturen von 60 °C und darunter eine verbesserte Bleichwirkung zu erreichen, können **Bleichaktivatoren** in die Präparate eingearbeitet werden. Beispiele hierfür sind mit Wasserstoffperoxid organische Persäuren bildende N-Acyl- bzw. O-Acyl-Verbindungen, vorzugsweise N,N'-tetraacylierte Diamine, ferner Carbonsäureanhydride und Ester von Polyolen wie Glucosepentaacetat. Der Gehalt der bleichmittelhaltigen Mittel an Bleichaktivatoren liegt in dem üblichen Bereich, vorzugsweise zwischen 1 und 10 Gew.-% und insbesondere zwischen 3 und 8 Gew.-%. Besonders bevorzugte Bleichaktivatoren sind N,N,N',N'-Tetraacetylethylendiamin und 1,5-Diacetyl-2,4-dioxo-hexahydro-1,3,5-triazin.

Als **Enzyme** kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Ihr Anteil kann etwa 0,2 bis etwa 2 Gew.-% betragen. Die Enzyme können an Trägerstoffen adsorbiert und/oder in Hüllsubstanzen eingebettet sein, um sie gegen vorzeitige Zersetzung zu schützen. Zusätzlich zu den mono- und polyfunktionellen Alkoholen und den Phosphonaten können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2-Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B4O₇).

**Vergrauungsinhibitoren** haben die Aufgabe, den von der Faser abgelösten Schmutz in der Flotte suspendiert zu halten und so das Vergrauen zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäwen der Stärke oder der Cellulose oder Salze von sauren Schwefelsäureestem der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw. Auch Polyvinylpyrrolidon ist brauchbar. Bevorzugt werden jedoch Celluloseether, wie Carboxymethylcellulose, Methylcellulose, Hydroxyalkylcellulose und Mischether, wie Methylhydroxyethylcellulose, Methylhydroxypropylcellulose, Methylcarboxymethylcellulose und deren Gemische sowie Polyvinylpyrrolidon, beispielsweise in Mengen von 0,1 bis 5 Gew.-%, bezogen auf die Mittel.

Die Mittel können als **optische Aufheller** Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Einheitlich weiße Granulate werden erhalten, wenn die Mittel außer den üblichen Aufhellern in üblichen Mengen, beispielsweise zwischen 0,1 und 0,5 Gew.-%, vorzugsweise zwischen 0,1 und 0,3 Gew.-%, auch geringe Mengen, beispielsweise 10⁻⁶ bis 10⁻³ Gew.-%, vorzugsweise um 10⁻⁵ Gew.-%, eines blauen Farbstoffs enthalten. Ein besonders bevorzugter Farbstoff ist Tinolux® (Handelsprodukt der Ciba-Geigy).

Als schmutzabweisende Polymere (**"soil repellants**") kommen solche Stoffe in Frage, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt insbesondere im Bereich von 750 bis 5.000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5.000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymere, die verknüpfende Polyethylenglycoleinheiten mit einem Molekulargewicht von 750 bis 5.000, vorzugsweise von 1.000 bis etwa 3.000 und ein Molekulargewicht des Polymeren von etwa 10.000 bis etwa 50.000 aufweisen. Beispiele für handelsübliche Polymere sind die Produkte Milease® T (ICI) oder Repelotex® SRP 3 (Rhöne-Poulenc).

Beim Einsatz in maschinellen Waschverfahren kann es von Vorteil sein, den Mitteln übliche **Schauminhibitoren** zuzusetzen. Hierfür eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, z.B. solche aus Silikonen, Paraffinen oder Wachsen. Vorzugsweise sind die Schauminhibitoren, insbesondere silikon- oder paraffinhaltige Schauminhibitoren, an eine granulare, in Wasser lösliche bzw. dispergierbare Trägersubstanz gebunden. Insbesondere sind dabei Mischungen aus Paraffinen und Bistearylethylendiamiden bevorzugt.

### Beispiel

Analog der Offenbarung zur **WO 94/18303** wurde eine C12-Alkylbenzolsulfonsäure über eine Mehrstoffdüse mit einer 50 Gew.-%igen wässrigen Natronlauge sprühneutralisiert (Treibgas: Luft) und direkt in einer Anlage zur Granuliertrocknung (AGT) der Firma Glatt, Bundesrepublik Deutschland, zusammen mit Natriumsulfat granuliert und gleichzeitig getrocknet. Über eine Feststoffdosierung wurde das Natriumsalz der Toluolsulfonsäure unterhalb der Mehrstoffdüse, aber oberhalb des sich ausbildenden Wirbelbettes eingetragen. Als Startmasse wurde ein Tensidgranulat eingesetzt, das bei einem vorangegangenen Ansatz unter den selben Verfahrensbedingungen gewonnen worden war und in etwa dieselbe Zusammensetzung besaß wie die fertigen Granulate des Beispiels.

Der Durchsatz der Wirbelschicht wurde so gesteuert, daß Granulate der folgenden Zusammensetzung erhalten wurden:

| | |
|---|---|
| 82,5 Gew.-% | C12-Alkylbenzolsulfonat, Natriumsalz |
| 5,5 Gew.-% | Natriumcarbonat |
| 5,0 Gew.-% | Natriumsulfat |
| 5,0 Gew.-% | Toluolsulfonat, Natriumsalz |
| 2,0 Gew.-% | Wasser (Restmenge aus Rohstoffen). |

Das Natriumcarbonat wurde nicht zusätzlich zugesetzt, sondern bildete sich bei der Reaktion aus Verbrennungsgasen und Natronlauge.

Die Granulate hatten ein Schüttgewicht von 660 g/l und wiesen folgende Siebanalyse auf (in mm und Gew.-%):

| **Siebanalyse (%)** | | | | | | | |
|---|---|---|---|---|---|---|---|
| mm | > 1,6 | > 1,25 | > 0,8 | > 0,4 | > 0,2 | > 0,1 | < 0,1 |
| Gew.-% | 0,1 | 14,7 | 54,7 | 28,5 | 2,0 | 0,1 | 0,0 |

Die Granulate waren staubfrei und klebten nicht.

## Patentansprüche

1. Nichtklebrige, staubfreie Alkylbenzolsulfonat-Granulate, wobei die Alkylbenzolsulfonate 9 bis 18 Kohlenstoffatomen im Alkylrest enthalten, enthaltend Toluolsulfonate, mit Aktivsubstanzgehalten von mindestens 60 Gew.-% und Schüttgewichten über 550 g/l **dadurch gekennzeichnet, dass** sie zu mindestens 50 Gew.-% nach der Siebanalyse einen Teilchendurchmesser von mindestens 0,8 mm aufweisen und die Kornverteilung der Granulate nach der Siebanalyse wie folgt ist:
| Teilchengröße | prozentuale Zusammenseztung in Gew.-% |
|---|---|
| unter 0,4 mm | 0 - 3 % |
| zwischen 0,4 und 0,8 mm | 22 - 35 % |
| zwischen 0,8 mm und 1,25 mm | 40 - 60 % |
| zwischen 1,25 und 1,6 mm | 10 - 25 % |
| über 1,6 mm | 0 - 0,5 % . |

2. Alkylbenzolsulfonat-Granulate nach Anspruch 1, **dadurch gekennzeichnet, dass** sie die Toluolsulfonate in Mengen von 3 bis 10 Gew.-% - bezogen auf Granulat - enthalten.

3. Alkylbenzolsulfonat-Granulate nach einem der Ansprüche 1 oder 2, **dadurch gekennzeichnet, dass** sie die Alkylbenzolsulfonate in Mengen von 75 bis 85 Gew.-% - bezogen auf Granulat - enthalten.

4. Alkylbenzolsulfonat-Granulate nach einem der Ansprüche 1 bis 3, **dadurch gekennzeichnet, dass** sie in Mengen bis zu 5 Gew.-% - bezogen auf Granulat - Restmengen an Wasser enthalten.

5. Alkylbenzolsulfonat-Granulate nach einem der Ansprüche 1 bis 4, **dadurch gekennzeichnet, dass** sie ad 100 Gew.-% Alkalisalze, vorzugsweise Alkalicarbonate und/oder Alkalisulfate enthalten.

6. Feste Wasch-, Spül- und Reinigungsmittel enthaltend Alkylbenzolsulfonat-Granulate nach Anspruch 1.

## Claims

1. Non-tacky, dust-free alkyl benzenesulfonate granules - the alkyl benzenesulfonates containing 9 to 18 carbon atoms in the alkyl group - containing toluenesulfonates and having active substance contents of at least 60% by weight and bulk densities above 550 g/l, **characterized in that**, according to sieve analysis, at least 50% by weight of the granules have a particle diameter of at least 0.8 mm and the particle size distribution according to sieve analysis is as follows:
| particle size | percentage composition in % by weight |
|---|---|
| under 0.4 mm | 0 - 3% |
| between 0.4 and 0.8 mm | 22 - 35% |
| between 0.8 mm and 1.25 mm | 40 - 60% |
| between 1.25 and 1.6 mm | 10 - 25% |
| over 1.6 mm | 0 - 0.5% |

2. Alkyl benzenesulfonate granules as claimed in claim 1, **characterized in that** they contain the toluene sulfonates in quantities of 3 to 10% by weight, based on granules.

3. Alkyl benzenesulfonate granules as claimed in claim 1 or 2, **characterized in that** they contain the alkyl benzenesulfonates in quantities of 75 to 85% by weight, based on granules.

4. Alkyl benzenesulfonate granules as claimed in any of claims 1 to 3, **characterized in that** they contain residual water in quantities of up to 5% by weight, based on granules.

5. Alkyl benzenesulfonate granules as claimed in any of claims 1 to 4, **characterized in that** they contain alkali metal salts, preferably alkali carbonates and/or alkali metal sulfates, to 100% by weight.

6. Solid laundry detergents, dishwashing detergents and cleaning preparations containing the alkyl benzenesulfonate granules claimed in claim 1.

## Revendications

1. Granulés d'alkylbenzènesulfonates non collants, exempts de poussière, dans lesquels les alkylbenzènesulfonates contiennent de 9 à 18 atomes de carbone dans le reste alkyle, contenant des toluènesulfonates, ayant des teneurs en substance active d'au moins 60 % en poids et des densités apparentes de plus de 550 g/l,
**caractérisés en ce qu'**
ils présentent à raison d'au moins 50 % en poids, selon l'analyse par tamisage, un diamètre de particule d'au moins 0,8 mm et la distribution granulométrique des granulés selon l'analyse par tamisage est comme suit :
| Taille de particule | Composition en % en poids |
|---|---|
| inférieure à 0,4 mm | 0 - 3 % |
| entre 0,4 et 0,8 mm | 22 - 35 % |
| entre 0,8 mm et 1,25 mm | 40 - 60 % |
| entre 1,25 et 1,6 mm | 10 - 25 % |
| supérieure à 1,6 mm | 0 - 0,5 %. |

2. Granulés d'alkylbenzènesulfonates selon la revendication 1,
**caractérisés en ce qu'**
ils contiennent les toluènesulfonates en proportions de 3 à 10 % en poids, par rapport au granulé.

3. Granulés d'alkylbenzènesulfonates selon la revendication 1 ou 2,
**caractérisés en ce qu'**
ils contiennent les alkylbenzènesulfonates en proportions de 75 à 85 % en poids, par rapport au granulé.

4. Granulés d'alkylbenzènesulfonates selon l'une quelconque des revendications 1 à 3,
**caractérisés en ce qu'**
ils contiennent des quantités résiduelles d'eau en proportions allant jusqu'à 5 % en poids, par rapport au granulé.

5. Granulés d'alkylbenzènesulfonates selon l'une quelconque des revendications 1 à 4,
**caractérisés en ce qu'**
ils sont complètes à 100 % en poids par des sels alcalins, de préférence des carbonates de métaux alcalins et/ou des sulfates de métaux alcalins.

6. Produits de lavage, de rinçage et de nettoyage contenant des granulés d'alkylbenzènesulfonates selon la revendication 1.
